# FASCICULE DE BREVET EUROPEEN

(11) **EP 0 729 747 B1**
(45) Date de publication et mention de la délivrance du brevet: **07.05.1997**
(21) Numéro de dépôt: 96400305.7
(22) Date de dépôt: 14.02.1996
(51) Int. Cl.: A61K 7/48, A61K 7/00

(54) **Utilisation de composés amphiphiles en tant qu'agent épaississant de milieux non aqueux et composition obtenue**
Verwendung von amphiphilen Verbindungen als Verdickungsmittel in nichtwässrigen Medien und die so hergestellte Zusammensetzungen
Use of amphiphilic compounds as thickening agent of non aqueous media and composition obtained

(30) Priorité: 03.03.1995 FR 9502521
(43) Date de publication de la demande: 04.09.1996
(73) Titulaire: L'OREAL, 75008 Paris (FR)
(72) Inventeur: Ribier, Alain, Décédé (FR); Legret, Sylvie, F-92320 Chatillon (FR); Richart, Pascal, F-75013 Paris (FR)
(74) Mandataire: Lhoste, Catherine

(56) Documents cités:
- EP-A- 0 285 768
- WO-A-92/05764
- FR-A- 2 700 267
- SEIFEN-ÖLE-FETTE-WACHSE, vol. 114, no. 1, 1988, pages 7-9, XP002005196 "Zucker als Rohstoffbasis: Glucamine und Galaktamine finden Anwendungen im Körperpflegemittelbereich"
- LIQUID CRYSTALS, vol. 3, no. 11, 1988, pages 1569-1581, XP002005197 J.W. GOODBY: "The thermotropic liquid-crystalline properties of some straight chain carbohydrate amphiphiles"

## Description

L'invention se rapporte à l'utilisation, en tant qu'agent épaississant d'un milieu non aqueux ou anhydre, d'au moins un composé particulier de nature amphiphile naturel ou synthétique ainsi qu'à la composition épaissie obtenue. Cette dernière peut être utilisée principalement dans le domaine de la cosmétique, mais aussi dans les domaines de la pharmacie, des peintures et vernis, des lubrifiants, des combustibles et de l'industrie alimentaire.

L'épaississement des milieux non aqueux a toujours été un problème difficile à résoudre pour lequel différentes solutions ont été proposées. On sait réaliser des milieux non aqueux épaissis en employant des composés conférant au milieu sa rigidité. Ces composés ont souvent de nombreux inconvénients comme par exemples certaines incompatibilités avec les composants de la solution ou encore des effets indésirables sur la peau qui font que l'on ne peut les utiliser qu'en quantité restreinte lorsque l'on veut conserver un bon agrément cosmétique sur la peau.

On connaît depuis longtemps l'utilisation de gélifiants minéraux comme la silice ou l'argile, ou de gélifiants organiques comme les polysaccharrides. L'inconvénient majeur de ce genre de gélifiants réside dans le fait qu'ils donnent naissance à des gels fragiles, sensibles au cisaillement, opaques et peu cosmétiques.

ll a également été proposé d'utiliser des N-acylaminoacides comme ceux décrits dans la demande de brevet FR-A- 2281162, des sels d'acides gras, tels ceux d'aluminium, de magnésium ou de calcium comme ceux décrits dans le brevet US-A- 2789329, des esters glycérolés d'acides gras, des esters gras de dextrine.

Les dérivés d'acides aminés et les différents sels d'acides gras sont des molécules ioniques. Ils sont donc difficilement compatibles avec l'ensemble des différents adjuvants ioniques ou non ioniques que l'on utilise dans les milieux épaissis. Ceci a pour conséquence d'entraîner des incompatibilités de formulation. On note par exemple l'apparition de phénomènes de synérèse d'huile aprés conservation, notamment à température élevée.

On connaît également les propriétés épaississantes des esters glycérolés d'acides gras, des esters gras de dextrine, des colloïdes inorganiques tels que la bentone, ou bien encore des produits naturels comme la cire d'abeille ou la cire de carnauba. Ces produits nécessitent d'être utilisés à de fortes concentrations, ce qui entraîne une perte de cosméticité, comme par exemple l'apparition d'un effet collant notamment lorsqu' on utilise de la bentone.

De plus si on utilise des esters, ceux-ci sont généralement sous forme de mélange d'esters dont les proportions sont variables et souvent peu définies. Les esters présentent également une grande sensibilité au pH. On retrouve là encore des difficultés de formulation.

On peut également citer les dérivés non ioniques de synthèse, de la famille des acides 11-N-alkyloxycarbonylamino-undécanoïques et leurs esters, décrits dans la demande de brevet FR-A-2647445. Ces composés ne permettent pas l'obtention de solutions transparentes. De plus on constate une certaine instabilité des milieux épaissis les contenant, entraînant l'apparition de cristaux dans la solution épaissie.

A la connaissance de la demanderesse, il n'a jamais été possible avec les solutions techniques de l'art antérieur, de réaliser des solutions non aqueuses épaissies ayant une bonne stabilité en même temps qu'un bon agrément cosmétique après étalement sur la peau.

De plus, on sait que parmi les composants des couches supérieures de la peau, on trouve dans l'épiderme un ciment intercellulaire. Ce ciment intercellulaire lie entre-elles les cellules de la couche cornée ; il est responsable de la cohésion de ces cellules et il règle leur desquamation. De plus, il assure la majeure partie de la fonction barrière de la peau. Cette fonction n'est réellement efficace que lorsque, entre autres, l'intégrité de ce ciment est respectée.

Ce ciment intercellulaire est composé de feuillets lipidiques, conférant à celui-ci une structure lamellaire.

Par biomimétisme, toute structure lipidique lamellaire similaire peut venir se fondre dans le ciment intercellulaire et ainsi soit permettre sa restructuration, soit y délivrer des actifs.

Depuis les travaux de Bangham (Bangham et coll. J. Mol. Biol. 13, 238, 1965), on sait réaliser de telles structures avec des liquides aqueux. Ainsi prépare-t-on des liposomes qui sont des vésicules sphériques dont la cavité centrale aqueuse est entourée d'au moins un feuillet lipidique.

Ces structures sont largement utilisées car elles sont parfaitement assimilées par la peau et elles permettent de manière efficace la délivrance d'actifs dans celle-ci.

La plupart du temps, les compositions à base de liposomes ont une consistance proche de celle d'un lait ou d'une crème, parfaitement compatible avec l'usage auquel elles sont destinées.

Il est cependant parfois nécessaire que de telles compositions se présentent sous une forme plus solide ; c'est par exemple le cas lorsqu'on veut réaliser des bâtonnets. Ceci peut être réalisé avec des composés particuliers qui ne peuvent être utilisés qu'en quantité restreinte si l'on veut conserver un bon agrément cosmétique sur la peau.

A la connaissance de la demanderesse, il n'a jamais été possible avec les solutions techniques de l'art antérieur, de réaliser avec des solutions non aqueuses des structures feuilletées lamellaires donc proches de la structure du ciment intercellulaire des couches supérieures de la peau.

Un autre but de la présente invention est donc de proposer un milieu non aqueux épaissi présentant et/ou donnant à la composition le contenant une aptitude à pénétrer aisément dans les couches supérieures de la peau.

Ainsi, la demanderesse a pu montrer que le choix de certains composés amphiphiles particuliers, naturels ou synthétiques, dans des conditions particulières, permet outre l'épaississement des milieux non aqueux, la réalisation d'une solution non aqueuse épaissie présentant une structure de réseau mésomorphe, telle que celle des cristaux liquides.

Cet état mésomorphe, entre l'état cristallin et l'état liquide, permet notamment l'obtention de gels transparents.

Après de longues recherches, c'est de manière surprenante et inattendue que la demanderesse a pu montrer que le choix de certains composés amphiphiles particuliers, naturels ou synthétiques, permet de résoudre les problèmes rencontrés dans l'épaississement des milieux non aqueux.

L'invention a donc pour objet l'utilisation dans un milieu non aqueux, d'au moins un composé amphiphile choisi parmi :
le N-oléoyl-N-méthyl-glucamine ;
le N-2-butyloctyloxycarbonyl-N-méthyl-D-glucamine ;
le N-2-hexyldécyloxycarbonyl-N-méthyl-D-glucamine pour épaissir ledit milieu.

L'invention a aussi pour objet une composition anhydre contenant, dans un milieu non aqueux une quantité efficace d'un agent épaississant choisi parmi les composés précédents.

Le milieu non aqueux épaissi trouve une application notamment dans les domaines de la cosmétique, de la pharmacie, des peintures et vernis, des lubrifiants, des combustibles et de l'industrie alimentaire.

Ces composés amphiphiles présentent une propriété épaississante remarquable. Cette propriété a pour conséquence de permettre l'utilisation de faibles quantités de ce type de composé dans les milieux à épaissir.

L'utilisation de ces composés amphiphiles, seuls ou en mélange, permet d'obtenir des milieux épaissis ne présentant pas les désavantages connus, conférés par les produits de l'art antérieur. On notera en particulier que les milieux ainsi épaissis présentent un bon agrément cosmétique après étalement sur la peau, conférant en particulier un effet de douceur très apprécié.

L'invention a plus particulièrement pour objet l'utilisation dans un milieu non aqueux, en tant qu'agent épaississant, d'au moins un composé amphiphile choisi parmi les composés ci-dessus. Pour réaliser un milieu non aqueux épaissi présentant une structure de réseau mésomorphe.

On peut ainsi obtenir une structure lamellaire, composée de feuillets, similaire à celle du ciment intercellulaire des couches supérieures de la peau.

Par biomimétisme, cette structure peut venir se fondre dans le ciment intercellulaire et ainsi permettre sa restructuration ou y délivrer des actifs.

Ces structures sont parfaitement assimilables par la peau et elles permettent, de manière efficace, la délivrance d'actifs dans celle-ci.

A ce titre, le milieu non aqueux épaissi peut par lui même être actif ou permettre le transport d'un ou plusieurs actifs notamment cosmétique ou dermatologique.

Un des grands avantages de l'invention réside donc dans le fait qu'il est désormais possible d'obtenir des milieux non aqueux épaissis en réseaux cristallins liquides présentant au niveau de la peau une bonne absorption et un bon agrément.

L'utilisation de l'invention en cosmétique apparaît donc comme très avantageuse.

Le milieu ainsi épaissi présente en outre une homogénéité stable dans le temps.

On peut moduler sa viscosité pour obtenir des milieux épaissis dont la consistance peut varier de l'état fluide à un état semi-solide idéal pour la préparation de bâtonnets rigides.

Bien entendu, les quantités de composés dans le milieu à épaissir dépendent du degré d'épaississement que l'on désire obtenir.

A titre indicatif, les composés peuvent être utilisés à des concentrations comprises dans la gamme allant de 0,1 % à 20 %, et de préférence de 0,5 % à 10 % en poids par rapport au poids total du milieu.

On peut obtenir un milieu épaissi qui a la propriété d'être structuré sous forme d'un réseau mésomorphe cristal-liquide discontinu en utilisant une quantité du composé amphiphile suffisante, en général allant de 0,1 % à 2 %.

La structure mésomorphe cristal-liquide continue communément appelée "gel", car de forme gélifiée, s'obtient généralement en utilisant une quantité du composé amphiphile supérieure ou égale à 2 % et de préférence de 2% à 8% du poids total de la composition.

Le milieu épaissi ainsi réalisé est tel que son utilisation est aisée. En particulier, il ne coule pas, il présente une bonne prise au doigt ou une rigidité suffisante pour constituer des bâtonnets rigides, et il est confortable au toucher.

Le milieu non aqueux peut être constitué d'au moins un liquide huileux, un alcool gras ou un solvant dans lequel peut être dissous au moins un actif. Par milieu non aqueux, on peut comprendre un milieu contenant moins de 5% d'eau.

Parmi les liquides huileux, on peut citer les supports huileux habituellement utilisés en cosmétique, et les liquides huileux pouvant être actifs par eux mêmes.

Ainsi, on peut citer les supports huileux tels que les triglycérides, les huiles, es gommes ou encore les huiles actives par elles-mêmes, comme par exemple les vitamines E et leur dérivés, les filtres solaires, les émollients comme le perhydrosqalène, l'huile balance (triglycérides riches en acides gras essentiels) ou les dérivés de farnesol (Unibovit B-33 de la société lnduchem).

Parmi les alcools gras, on peut citer les alcools cétylique et stéarique.

Parmi les solvants, on peut citer l'octyldodécanol (Eutanol G de la société Henkel) ou l'oléate de décyle.

L'invention permet en outre d'incorporer de grandes quantités d'actifs.

Il est ainsi possible d'incorporer sans difficultés dans le milieu ainsi épaissi des actifs lipophiles particulièrement hydrophobes.

Quand le milieu non aqueux épaissi selon l'invention n'est pas actif par lui-même, la quantité d'actifs que l'ont peut incorporer dépend de sa solubilité dans les huiles supports, alcools gras ou solvants.

On citera par exemple, comme actifs lipophiles la vitamine A et ses dérivés de type esters ou alcools, les lipohydroxyacides comme par exemple l'acide N-octanoyl-5-salicylique, les glycérides de vitamines F, les filtres U.V. comme par exemple le Parsol.

L'épaississement du milieu peut s'effectuer par solubilisation complète du composé amphiphile dans le milieu non aqueux par un échauffement à une température comprise entre 70°C et 90°C, puis en laissant reposer le mélange jusqu'à ce qu'il soit totalement épaissi.

Le milieu ainsi épaissi peut constituer ou être un constituant de compositions épaissies cosmétiques ou pharmaceutiques, de peintures et de vernis, de lubrifiants, de combustibles ou encore de produits alimentaires.

En cosmétique ou en pharmacie, ce milieu épaissi peut, entre autres, se présenter sous forme de sticks, de laits ou de vernis.

La composition comprend alors, les ingrédients habituellement utilisés en cosmétique ou en pharmacie. Ainsi, elle peut comprendre notamment au moins un additif choisi parmi les alcools gras, les épaississants, les esters d'acides gras, les esters d'acides gras et de glycérol, les silicones (volatiles ou non, fonctionnalisés ou non), les tensioactifs, les parfums, les conservateurs, les filtres solaires, les protéines, les vitamines, les polymères, les huiles organiques ou inorganiques.

Tous ces milieux épaissis sont préparés selon les méthodes usuelles connues de l'homme de l'art.

On va maintenant donner à titre d'illustration des exemples de divers milieux non aqueux épaissis par les composés amphiphiles selon l'invention. Ces exemples ne sauraient limiter en aucune façon la portée de l'invention.

### EXEMPLES

### Exemple 1 :

On va maintenant donner à titre d'illustration les résultats d'essais comparatifs des effets des composés retenus selon l'invention et de composés proches des mêmes familles chimiques, réalisés sur un milieu non aqueux constitué d'acétate d'α tocophérol (actif huileux).

Les différents composés testés ont été introduits au taux de 3% en poids par rapport au poids total du milieu épaissi obtenu.
- Composés testés :
   A : N-oléoyl-N-méthyl-glucamine
   B : N-hexadécyloxycarbonyl-N-méthyl-glucamine
   C : N-docosanoyl-N-méthyl-glucamine
   D : N-2-hexyldécyloxycarbonyl-N-méthyl-glucamine
   E : N-2-décyltétradécyloxycarbonyl-N-méthyl-glucamine
   F : N-2-dodécylhexadécyloxycarbonyl-N-méthyl-glucamine
   G : N-2-butyloctyloxycarbonyl-N-méthyl-glucamine
   H : N-(2-butyl-octyloxycarbonyl)-aminopropanediol
   I : N-(butyl-2-octyloxycarbonyl)-glucamine
   J : N-(2-butyloctyloxycarbonyl)-éthanolamine
- Résultats :
   Les composés A, D et G, composés de l'invention, conduisent à l'obtention de gels transparents.
   Les composés B, C, E, F, H, I, et J conduisent à l'obtention de solutions opaques non gélifiées présentant un état cristallin au microscope.

Seuls les composés de l'invention conduisent à la solution recherchée, c'est-à dire l'obtention d'un milieu non aqueux épaissi.

### Exemple 2 : fard à paupières :

| | |
|---|---|
| N-2-butyloctyloxycarbonyl-N-méthyl-D-glucamine | 3,0 % |
| Acétate de D,L alpha tocophéryle | 15,0 % |
| D-alpha tocophérol | 3,0 % |
| Unibiovit B 33 * | 3,0 % |
| Huile balance ** | 15,0 % |
| Cyclopentadiméthylsiloxane | 20 % |
| Q2-140 *** | 39,8 % |
| Palmitate de rétinyle **** | 0,2 % |

| | |
|---|---|
| * : Mélange de farnesol, acétate de farnesyle, linoléate d'éthyle dans oléate de décyle, vendu par la société Induchem. | |
| ** : Mélange d'huiles végétales stabilisé, vendu par la société Nestlé. | |
| *** : Mélange de polydiméthylsiloxane-ol et polydiméthylsiloxane, vendu parla société Dow Corning. | |
| **** : Dans huile d'arachide à 55 % stabilisée. | |

Après mélange des différents ingrédients, on obtient un fard à paupières épaissi présentant de bonnes qualités d'étalement et un touché cosmétique particulièrement agréable.

### Exemple 3 : Milieu épaissi anti-cerne

| | |
|---|---|
| N-2-butyloctyloxycarbonyl-N-méthyl-D-glucamine | 3,0 % |
| Acétate de D,L alpha tocophéryle | 15,0 % |
| D-alpha tocophérol | 3,0 % |
| Unibiovit B 33 * | 0,5 % |
| Huile balance ** | 5,0 % |
| Triglycéride d'acide caprilique-caprique | 10,0 % |
| Cyclopentadiméthylsiloxane | 22,42 % |
| Silicone stabiliseur TP.232 *** | 40,0 % |
| Santoquin **** | 0;03 % |
| Parsol MCX⁵ * | 1,0 % |
| Acide citrique 1 H₂O | 0,05 % |

| | |
|---|---|
| * : Mélange de farnesol, acétate de farnesyle, linoléate d'éthyle dans oléate de décyle, vendu par la société Induchem. | |
| ** : Mélange d'huiles végétales stabilisé vendu par la société Nestlé. | |
| *** : Polydiméthylsiloxane à haut poids moléculaire dans cyclopentadiméthylsiloxane (15/85), vendu par la société OSI. | |
| **** : Ethoxy-6 dihydro-1,2 triméthyl-2,2,4 quinoline, vendu par la société Monsanto. | |
| 5 * : P-méthoxy-4 cinnamate d'éthyl-hexyle stabilisé, vendu par la société Givaudan Roure. | |

Après mélange des différents ingrédients, on obtient un milieu épaissi anti-cerne doux au touché.

### Exemple 4 : gel protecteur antisolaire

| | |
|---|---|
| N-2-butyloctyloxycarbonyl-N-méthyl-D-glucamine | 3,0 % |
| Acétate de D,L alpha tocophéryle | 15,0 % |
| D-alpha tocophérol | 3,0 % |
| Huile balance IA stabilisée * | 5,0 % |
| Triglycéride d'acide caprilique-caprique | 10,0 % |
| Dodécaméthyl cyclohexasiloxane ** | 22,45 % |
| Silicone stabiliseur TP.232 *** | 40,0 % |
| Meroxyl SAD | 0,5 % |
| Parsol MCX **** | 1,0 % |

| | |
|---|---|
| * : Mélange d'huile de tournesol, hybride de tournesol, rosier muscat, pépin de cassis (31/60/5,95/3) stabilisé, vendu par la société Nestlé. | |
| ** : vendu par la société Dow Corning | |
| *** : Polydiméthylsiloxane à haut poids moléculaire dans cyclopentadiméthylsiloxane (15/85), vendu par la société OSI. | |
| **** : P-méthoxy-4 cinnamate d'éthyl-hexyle stabilisé, vendu par la société Givaudan Roure. | |

Après mélange des différents ingrédients, on obtient un gel agréable au touché, présentant de bonne qualité d'étalement.

### Exemple 5 : gel revitalisant

| | |
|---|---|
| N-2-butyloctyloxycarbonyl-N-méthyl-D-glucamine | 3,0 % |
| Acétate de D,L alpha tocophéryle | 15,0 % |
| D-alpha tocophérol | 3,0 % |
| Unibiovit B 33 * | 3,0 % |
| Huile balance ** | 15,0 % |
| Cyclopentadiméthylsiloxane | 20,0 % |
| Q2-140 *** | 40,3 % |
| Palmitate de rétinyle **** | 0,2 % |
| Acide N-octanoyl-5-salicylique | 0,5 % |

| | |
|---|---|
| * : Mélange de farnesol, acétate de farnesyle , linoléate d'éthyle dans oléate de décyle, vendu par la société Induchem. | |
| ** : Mélange d'huiles végétales stabilisé vendu par la société Nestlé. | |
| *** : Mélange de polydiméthylsiloxane-ol et polydiméthylsiloxane, vendu par la société Dow Corning. | |
| **** : Dans huile d'arachide à 55 % stabilisée. | |

Après mélange des différents ingrédients, on obtient un gel transparent.

## Revendications

1. Utilisation, dans un milieu non aqueux, d'au moins un composé amphiphile choisi parmi :
le N-oléoyl-N-méthyl-glucamine ;
le N-2-butyloctyloxycarbonyl-N-méthyl-D-glucamine ;
le N-2-hexyldécyloxycarbonyl-N-méthyl-D-glucamine ; pour épaissir ledit milieu.

2. Utilisation selon la revendication précédente, caractérisée en ce que le milieu non aqueux épaissi présente une structure de réseau mésomorphe.

3. Utilisation selon l'une quelconque des revendications précédentes, caractérisée en ce que la quantité du composé amphiphile est comprise dans la gamme allant de 0,1 % à 20 % en poids par rapport au poids total du milieu épaissi.

4. Utilisation selon l'une quelconque des revendications précédentes, caractérisée en ce que la quantité du composé amphiphile est comprise dans la gamme allant de 0,5 % à 10 % en poids par rapport au poids total du milieu épaissi.

5. Utilisation selon la revendication 2, caractérisée en ce que la quantité du composé amphiphile est suffisante pour obtenir une structure sous forme d'un réseau mésomorphe cristal-liquide discontinu.

6. Utilisation selon la revendication 5, caractérisée en ce que la quantité du composé amphiphile est comprise dans la gamme allant de 0,1% à 2 % en poids par rapport au poids total du milieu épaissi.

7. Utilisation selon la revendication 2, caractérisée en ce que la quantité du composé amphiphile est suffisante pour obtenir une structure sous forme d'un réseau mésomorphe cristal-liquide continu.

8. Utilisation selon la revendication 7, caractérisée en ce que la quantité du composé amphiphile est supérieure à 2 % en poids par rapport au poids total du milieu épaissi.

9. Utilisation selon la revendication 8, caractérisée en ce que la quantité du composé amphiphile est choisi dans la gamme allant de 2% à 8% du poids total du milieu épaissi.

10. Utilisation selon l'une quelconque des revendications 1 à 9, caractérisée en ce que le milieu non aqueux est constitué d'un liquide huileux, d'un alcool gras ou d'un solvant.

11. Utilisation selon la revendication précédente, caractérisée par le fait que le liquide huileux est choisi parmi les triglycérides, les huiles et les gommes ou encore les huiles actives par elles-mêmes.

12. Utilisation selon la revendication 11, caractérisée en ce que les huiles actives sont choisies parmi les vitamines E et leur dérivés, les filtres solaires, les émollients comme le perhydrosqalène, l'huile balance ou les dérivés de farnesol.

13. Utilisation selon la revendication 9, caractérisée par le fait que l'alcool gras est choisi parmi les alcools cétylique et stéarique.

14. Utilisation selon la revendication 9, caractérisée par le fait que le solvant est choisi parmi l'octyldodécanol et l'oléate de décyle.

15. Procédé d'épaississement d'un milieu non aqueux, caractérisé en ce que l'on solubilise au moins un composé amphiphile choisi parmi le N-oléoyl-N-méthyl-glucamine, le N-2-butyloctyloxycarbonyl-N-méthyl-D-glucamine, le N-2-hexyldécyloxycarbonyl-N-méthyl-D-glucamine dans un milieu non aqueux par chauffage à une température comprise dans la gamme allant de 70°C à 90°C, puis que l'on laisse reposer le mélange jusqu'à ce qu'il soit totalement épaissi.

16. Utilisation du milieu non aqueux épaissi obtenu selon l'une quelconque des revendications 1 à 14 dans des compositions cosmétiques ou pharmaceutiques, des peintures, des vernis, des lubrifiants, des combustibles ou encore des produits alimentaires.

17. Composition anhydre contenant dans un milieu non aqueux une quantité efficace d'un agent épaississant choisi parmi :
le N-oléoyl-N-méthyl-glucamine ;
le N-2-butyloctyloxycarbonyl-N-méthyl-D-glucamine ;
le N-2-hexyldécyloxycarbonyl-N-méthyl-D-glucamine ;

18. Composition selon la revendication 17, caractérisée en ce que la quantité d'agent épaississant est suffisante pour que le milieu épaissi présente une structure de réseau mésomorphe.

19. Composition selon la revendication 17 ou 18, caractérisée en ce que la quantité d'agent épaississant est suffisante pour que le milieu soit un gel transparent.

20. Composition selon l'une des revendications 17 à 19, caractérisée en ce que la quantité d'agent épaississant est suffisante pour que le milieu ait une structure de réseau mésomorphe cristal-liquide continu.

21. Composition selon l'une des revendications 17 à 20, caractérisée en ce que la quantité d'agent épaississant est choisie de 2 à 8% du poids total de la composition.

22. Composition selon l'une des revendications 17 à 21, caractérisée en ce que le milieu non aqueux est un milieu huileux.

23. Composition selon l'une des revendications 17 à 22, caractérisée en ce que le milieu est un milieu cosmétique ou pharmaceutique.

## Claims

1. Use, in a non-aqueous medium, of at least one amphiphilic compound chosen from:
N-oleoyl-N-methylglucamine;
N-2-butyloctyloxycarbonyl-N-methyl-D-glucamine;
N-2-hexyldecyloxycarbonyl-N-methyl-D-glucamine; in order to thicken the said medium.

2. Use according to the preceding claim, characterized in that the thickened non-aqueous medium exhibits a mesomorphic network structure.

3. Use according to either of the preceding claims, characterized in that the amount of the amphiphilic compound is in the range from 0.1% to 20% by weight with respect to the total weight of the thickened medium.

4. Use according to any one of the preceding claims, characterized in that the amount of the amphiphilic compound is in the range from 0.5% to 10% by weight with respect to the total weight of the thickened medium.

5. Use according to Claim 2, characterized in that the amount of the amphiphilic compound is sufficient to obtain a structure in the form of a non-continuous liquid-crystal mesomorphic network.

6. Use according to Claim 5, characterized in that the amount of the amphiphilic compound is in the range from 0.1% to 2% by weight with respect to the total weight of the thickened medium.

7. Use according to Claim 2, characterized in that the amount of the amphiphilic compound is sufficient to obtain a structure in the form of a continuous liquid-crystal mesomorphic network.

8. Use according to Claim 7, characterized in that the amount of the amphiphilic compound is greater than 2% by weight with respect to the total weight of the thickened medium.

9. Use according to Claim 8, characterized in that the amount of the amphiphilic compound is chosen in the range from 2% to 8% of the total weight of the thickened medium.

10. Use according to any one of Claims 1 to 9, characterized in that the non-aqueous medium is composed of an oily liquid, of a fatty alcohol or of a solvent.

11. Use according to the preceding claim, characterized in that the oily liquid is chosen from triglycerides, oils and gums, or alternatively oils which are active in themselves.

12. Use according to Claim 11, characterized in that the active oils are chosen from vitamins E and their derivatives, sunscreens, emollients, such as perhydrosqualene, balance oil or farnesol derivatives.

13. Use according to Claim 9, characterized in that the fatty alcohol is chosen from cetyl or stearyl alcohol.

14. Use according to Claim 9, characterized in that the solvent is chosen from octyldodecanol and decyl oleate.

15. Process for thickening a non-aqueous medium, characterized in that at least one amphiphilic compound chosen from N-oleoyl-N-methylglucamine, N-2-butyloctyl-oxycarbonyl-N-methyl-D-glucamine or N-2-hexyldecyloxy-carbonyl-N-methyl-D-glucamine is dissolved in a non-aqueous medium by heating to a temperature in the range from 70°C to 90°C and then in that the mixture is allowed to stand until it has completely thickened.

16. Use of the thickened non-aqueous medium obtained according to any one of Claims 1 to 14 in cosmetic or pharmaceutical compositions, paints, varnishes, lubricants, fuels or alternatively foodstuffs.

17. Anhydrous composition containing, in a non-aqueous medium, an effective amount of a thickening agent chosen from:
N-oleoyl-N-methylglucamine;
N-2-butyloctyloxycarbonyl-N-methyl-D-glucamine;
N-2-hexyldecyloxycarbonyl-N-methyl-D-glucamine.

18. Composition according to Claim 17, characterized in that the amount of thickening agent is sufficient for the thickened medium to exhibit a mesomorphic network structure.

19. Composition according to Claim 17 or 18, characterized in that the amount of thickening agent is sufficient for the medium to be a transparent gel.

20. Composition according to one of Claims 17 to 19, characterized in that the amount of thickening agent is sufficient for the medium to have a continuous liquid-crystal mesomorphic network structure.

21. Composition according to one of Claims 17 to 20, characterized in that the amount of thickening agent is chosen from 2 to 8% of the total weight of the composition.

22. Composition according to one of Claims 17 to 21, characterized in that the non-aqueous medium is an oily medium.

23. Composition according to one of Claims 17 to 22, characterized in that the medium is a cosmetic or pharmaceutical medium.

## Patentansprüche

1. Verwendung mindestens einer amphiphilen Verbindung, die ausgewählt ist unter
N-Oleoyl-N-methyl-glucamin;
N-2-Butyloctyloxycarbonyl-N-methyl-D-glucamin; und
N-2-Hexyldecyloxycarbonyl-N-methyl-D-glucamin
in einem nicht-wäßrigen Medium zur Verdickung dieses Mediums.

2. Verwendung nach dem vorstehenden Anspruch, dadurch gekennzeichnet, daß das verdickte, nicht-wäßrige Medium eine mesomorphe Netzstruktur aufweist.

3. Verwendung nach einem der vorstehenden Ansprüche, dadurch gekennzeichnet, daß die Menge der amphiphilen Verbindung im Bereich von 0,1 bis 20 Gew.-%, bezogen auf das Gesamtgewicht des verdickten Mediums, liegt.

4. Verwendung nach einem der vorstehenden Ansprüche, dadurch gekennzeichnet, daß die Menge der amphiphilen Verbindung im Bereich von 0,5 bis 10 Gew.-%, bezogen auf das Gesamtgewicht des verdickten Mediums, liegt.

5. Verwendung nach Anspruch 2, dadurch gekennzeichnet, daß die Menge der amphiphilen Verbindung ausreicht, um eine Struktur in Form eines diskontinuierlichen, flüssigkristallinen, mesomorphen Netzes zu erhalten.

6. Verwendung nach Anspruch 5, dadurch gekennzeichnet, daß die Menge der amphiphilen Verbindung im Bereich von 0,1 bis 2 Gew.-%, bezogen auf das Gesamtgewicht des verdickten Mediums, liegt.

7. Verwendung nach Anspruch 2, dadurch gekennzeichnet, daß die Menge der amphiphilen Verbindung ausreicht, um eine Struktur in Form eines kontinuierlichen, flüssigkristallinen, mesomorphen Netzes zu erhalten.

8. Verwendung nach Anspruch 7, dadurch gekennzeichnet, daß die Menge der amphiphilen Verbindung mehr als 2 Gew.-%, bezogen auf das Gesamtgewicht des verdickten Mediums, beträgt.

9. Verwendung nach Anspruch 8, dadurch gekennzeichnet, daß die Menge der amphiphilen Verbindung im Bereich von 2 bis 8 Gew.-% des gesamten verdickten Mediums gewählt wird.

10. Verwendung nach einem der Ansprüche 1 bis 9, dadurch gekennzeichnet, daß das nicht-wäßrige Medium aus einer öligen Flüssigkeit, einem Fettalkohol oder aus einem Lösungsmittel gebildet ist.

11. Verwendung nach dem vorstehenden Anspruch, dadurch gekennzeichnet, daß die ölige Flüssigkeit unter Triglyceriden, Ölen und Gummen oder unter von sich aus aktiven Ölen ausgewählt wird.

12. Verwendung nach Anspruch 11, dadurch gekennzeichnet, daß die aktiven Öle unter den Vitaminen E und ihren Derivaten, Sonnenschutzmitteln, Emollentien, wie Perhydrosqualen , Ausgleichsöl oder Farnesolderivaten ausgewählt sind.

13. Verwendung nach Anspruch 9, dadurch gekennzeichnet, daß der Fettalkohol unter Cetylalkohol und Stearylalkohol ausgewählt ist.

14. Verwendung nach Anspruch 9, dadurch gekennzeichnet, daß das Lösungsmittel unter Octyldodecanol und Decyloleat ausgewählt ist.

15. Verfahren zum Verdicken eines nicht-wäßrigen Mediums, dadurch gekennzeichnet, daß man mindestens eine amphiphile Verbindung, die unter N-Oleoyl-N-methylglucamin, N-2-Butyloctyloxycarbonyl-N-methyl-D-glucamin und N-2-Hexyl-decyloxycarbonyl-N-methyl-D-glucamin ausgewählt ist, in einem nicht-wäßrigen Medium durch Erwärmen auf eine Temperatur im Bereich von 70 bis 90°C in Lösung bringt und anschließend das Gemisch ruhen läßt, bis es vollständig verdickt ist.

16. Verwendung des nach einem der Ansprüche 1 bis 14 erhaltenen verdickten, nicht-wäßrigen Mediums in kosmetischen oder pharmazeutischen Zusammensetzungen, Anstrichmitteln, Lacken, Schmiermitteln, Brennstoffen oder Nahrungsmittelprodukten.

17. Wasserfreie Zusammensetzung, enthaltend in einem nicht-wäßrigen Medium eine wirksame Menge eines Verdickungsmittels, das ausgewählt ist unter:
N-Oleoyl-N-methyl-glucamin;
N-2-Butyloctyloxycarbonyl-N-methyl-D-glucamin; und
N-2-Hexyldecyloxycarbonyl-N-methyl-D-glucamin.

18. Zusammensetzung nach Anspruch 17, dadurch gekennzeichnet, daß die Menge des Verdickungsmittels ausreicht, daß das verdickte Medium eine mesomorphe Netzstruktur aufweist.

19. Zusammensetzung nach Anspruch 17 oder 18, dadurch gekennzeichnet, daß die Menge des Verdickungsmittels ausreicht, daß das Medium die Form eines durchsichtigen Gels aufweist.

20. Zusammensetzung nach einem der Ansprüche 17 bis 19, dadurch gekennzeichnet, daß die Menge des Verdickungsmittels ausreicht, daß das Medium eine kontinuierliche, flüssigkristalline, mesomorphe Netzstruktur aufweist.

21. Zusammensetzung nach einem der Ansprüche 17 bis 20, dadurch gekennzeichnet, daß die Menge des Verdickungsmittels im Bereich von 2 bis 8 Gew.-%, bezogen auf das Gesamtgewicht der Zusammensetzung, ausgewählt ist.

22. Zusammensetzung nach einem der Ansprüche 17 bis 21, dadurch gekennzeichnet, daß es sich beim nicht-wäßrigen Medium um ein öliges Medium handelt.

23. Zusammensetzung nach einem der Ansprüche 17 bis 22, dadurch gekennzeichnet, daß es sich beim Medium um ein kosmetisches oder pharmazeutisches Medium handelt.
